# EUROPEAN PATENT APPLICATION

(11) **EP 3 413 050 A1**
(43) Date of publication of application: **12.12.2018**
(21) Application number: 17174895.7
(22) Date of filing: 08.06.2017
(51) Int. Cl.: G01N 33/68, G01N 33/558

(54) **IN VITRO METHOD FOR THE DETERMINATION OF NEURODEGENERATIVE DISEASES**

(71) Applicant: SALION GmbH, 82541 Münsing (DE)
(72) Inventor: STANGL, Manfred, 82054 Sauerlach (DE); MARZINZIG, Michael, 89075 Ulm (DE); ABENDROTH, Dietmar, 89275 Thalfingen (DE)
(74) Representative: Weickmann & Weickmann PartmbB

(57) **Abstract**

The present invention concerns an in vitro method for the determination of a neurodegenerative disease wherein separately from each other the content of kynurenine and kynurenic acid in a body fluid is determined and the quotient of the content of kynurenine to the content of kynurenic acid is calculated.

## Description

### Background of the invention

Chronic progressive neurodegenerative diseases, such as Alzheimer's disease (AD), Parkinson's disease (PD) and vascular dementia (VD) display an increasing prevalence in parallel with the ongoing aging of the population, and have therefore generated considerable recent research interest. Despite extensive studies on the background of neurodegenerative processes, the exact molecular basis remains still to be clarified. There is accumulating evidence that the innate immune response in the brain is mainly influenced by inflammatory processes.

Although these devastating diseases have a serious impact on the quality of life of the patients, their management is often challenging. Current therapies offer mostly only symptomatic relief and no neuroprotective therapy is available. The pathomechanisms of different neurodegenerative disorders share a number of common features. Excitotoxicity, neuroinflammation, a mitochondrial disturbance and oxidative stress have been implicated in both acute and chronic neurological disorders. A diagnostic method, which helps to define more precisely the disease, is therefore desirable.

WO 2014/177680 discloses a diagnostic method for neurodegenerative disorders. The levels of kynurenine in plasma and/or in saliva are compared with the average level of kynurenine measured in comparable individuals who are not affected by such neurodegenerative diseases.

### The field of the present invention

Neurodegenerative processes share some common features, which are not disease-specific. While there are still a number of details that await elucidation, there are several common mechanisms that are widely accepted; the role of mitochondrial disturbances, excitotoxicity, neuro-inflammation and oxidative stress appear evident.

Glutamate excitotoxicity has been implicated in the pathomechanisms of ischemic stroke, traumatic brain injury, and various neurodegenerative disorders.

AD was earlier thought to involve a distinct pathology, which can be clearly distinguished from vascular dementia (VD). However, in recent years, the role of a cerebrovascular dysfunction has been linked to the neurodegenerative process of AD, and vascular risk factors have attracted growing attention in connection with AD development and progression.

Overlaps between VD and AD have long been recognized, but in recent years a complete paradigm shift has begun, and AD has been suggested to be a primarily vascular disease. Only a small proportion of AD cases have a genetic origin; the majorities are sporadic. The most important risk factor for the development of AD is advancing age, the prevalence and incidence data demonstrating an increasing tendency with rising age. Again to be mentioned, kynurenine plays a major role in vascular regulatory processes.

Similarly, an impaired cerebral blood flow and autoregulation capacity has been observed in animal models of AD, this impairment proving to be associated with oxidative stress. These findings link the presence of Aß to oxidative stress and neuroinflammation. Today under the new view of innate immune responses we would state that there is an activation of the innate inflammatory response accompanying this disease. In this theory the Aß molecule could have the role of an alarmin - same like ATP in other diseases - and is responsible for the activation of the inflammation via NALP-3-inflammasome. Second view is the generation of oxygen - radicals under a minimized blood flow.

### Summary of the present invention

It is an object to provide a diagnostic in vitro method and means for performing the diagnostic method that help to diagnose whether a person suffers from a neurodegenerative disease. Moreover, it is an object to differentiate neurodegenerative diseases from other impairments of mental capabilities that may be caused by other reasons. In addition the in vitro method of the present invention allows for therapy control in neurodegenerative diseases. The in vitro method of the present invention is performed outside the human body and there is no need that the test is performed in the presence of a medical doctor.

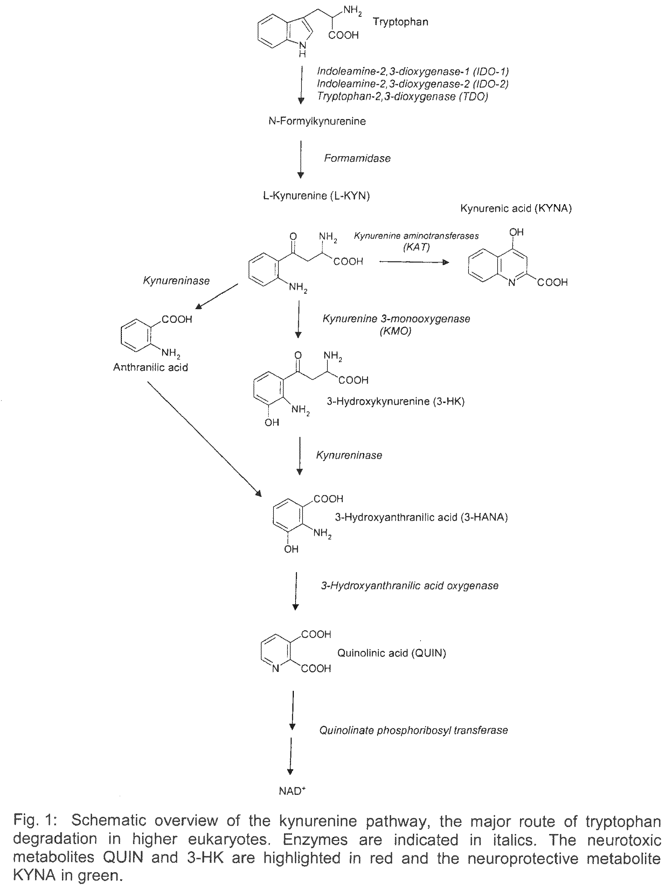

In the following the potential role of the kynurenine pathway (KP) in neurodegenerative diseases and its modulation for diagnostic purposes will be explained in more detail:
The present invention relates to the kynurenine pathway. Tryptophan is an essential amino acid that can be metabolized through different pathways, a major route being the kynurenine pathway. This pathway is illustrated in Figure 1. The first enzyme of the pathway, indolamine-2,3-dioxygenase (IDO-1) is strongly stimulated by inflammatory molecules, particularly interferon-γ. Thus, the kynurenine pathway is often systematically up-regulated when the immune response is activated. The biological significance is that on the one hand the depletion of tryptophan and generation of kynurenines play a key modularly role in the immune response. On the other hand it was found surprisingly that the level of kynurenine and kynurenic acid measured in the saliva could be used for the detection of a potential neurodegenerative disease that can otherwise not be easily detected. This is particularly surprising since neurodegenerative diseases relate to the brain that is separated from the rest of the body by the blood/liquor barrier.

The activation of indole amine 2,3-dioxygenase (IDO I), the main enzyme involved in the catabolism of tryptophan, generates immunosuppressive metabolites which counter-regulates immune activation.

Today it is known that the endothelium, once considered to be relative inert, is involved in various functions such as fibrinolysis, coagulation, vascular tone, growth and immune response. The most common reaction in the human body might be seen in the inflammatory response mediated by the innate immunity.

Indole amine 2,3 dioxygenase (IDO), an IFN-γ-inducible intracellular enzyme, catalyzes the first and rate-limiting step in the degradation of the essential amino acid tryptophan in the kynurenine pathway. The immunmodulatory effects of IDO are represented by the prevention of T cell proliferation, promotion of T cell apoptosis, induction of T cell ignorance, anergy, and generation of T regulatory cells. While IDO emerges as a regulator of immunity, its role in controlling allo-response is unfolding.

The KP is the main metabolic route of tryptophan (TRP) degradation in mammals; it is responsible for more than 95 % of the TRP catabolism in the human brain. The metabolites produced in this metabolic cascade, termed kynurenines, are involved in a number of physiological processes, including neurotransmission and immune responses. The KP also involves neurotoxic and neuroprotective metabolites, and alterations in their delicate balance have been demonstrated in multiple pathological processes.

The central intermediate of the KP is L-kynurenine (L-KYN), where the metabolic pathway divides into two different branches. L-KYN is transformed to either the neuroprotective kynurenic acid (KYNA) via kynurenine aminotransferase (KAT) or 3-hydroxy-L-kynurenine (3-OH-KYN), which is further metabolized in a sequence of enzymatic steps to yield finally NAD⁺ (as shown in Fig.1).

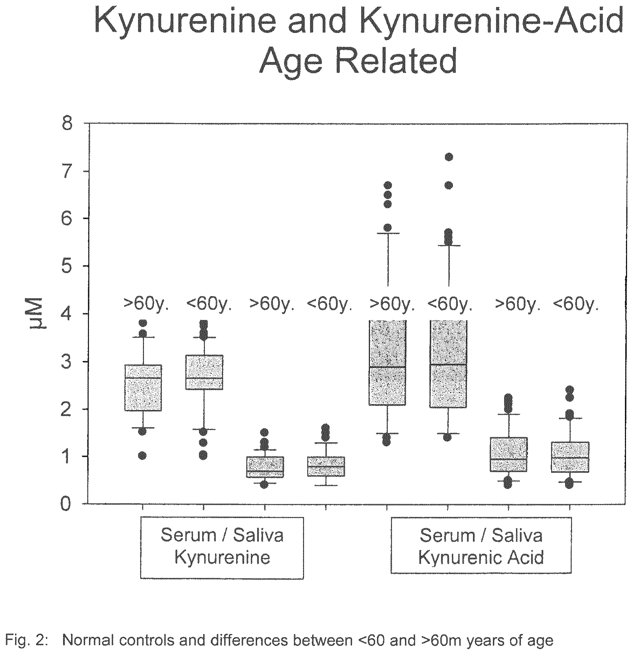

Imbalances in the KP are not only relevant in AD, but also in other disorders in which there is a cognitive decline, and influencing this delicate balance may be of utmost therapeutic value. Fig. 2 shows the relationship of kynurenine and kynurenine acid in healthy control persons whereby cohorts of volunteers over 60 years old and cohorts under 60 years were tested. Fig. 2 shows that the mean values of kynurenine and kynurenic acid in cohorts of healthy volunteers are very similar regardless whether the volunteers have an age above or below 60 years.

Changes in kynurenine metabolites have additionally been suggested to correlate with the infarct volume, the mortality of stroke patients and the post-stroke cognitive impairment.

In another study, serum kynurenine levels and inflammatory markers were measured in patients undergoing cardiac surgery; the results indicated an association of several kynurenine metabolite levels with the post-surgical cognitive performance.

The results of the present work show increased levels of tryptophan with decreased levels of kynurenine, anthranilic acid and 3-hydroxyanthranilic acid associated with bypass surgery, and a later increase in kynurenic acid. Levels of neopterine and lipid peroxidation products rose after surgery in non-bypass patients whereas TNF-α and S100B levels increased after bypass. Changes of neopterine levels were greater after non-bypass surgery. Cognitive testing showed that the levels of tryptophan, kynurenine, kynurenic acid and the kynurenine/tryptophan ratio, correlated with aspects of post-surgery cognitive function, and were significant predictors of cognitive performance in tasks sensitive to frontal executive function and memory. Thus, anesthesia and major surgery are associated with inflammatory changes (activation of the innate immune response according to generation of free radicals) and alterations in tryptophan oxidative metabolism that predict, and may play a role in, post-surgical cognitive function.

KP metabolites have also been implicated in vascular cognitive impairment. As concerns AD, a substantial amount of evidence demonstrates an altered tryptophan metabolism.

From the aspect of the peripheral kynurenine metabolism, decreased KYNA levels were measured in the serum, red blood cells and CSF of AD patients. Additionally, enhanced IDO activity was demonstrated in the serum of AD patients, as reflected by an increased KYN/TRP ratio, this elevation exhibiting inverse correlation with the rate of cognitive decline. IDO activation was also correlated with several immune markers in the blood, thereby indicating an immune activation, which lends further support to the role of neuroinflammation in the pathomechanism of AD. An increased IDO activity was also confirmed by immunohistochemistry in the hippocampus of AD patients, together with an enhanced QUIN immunoreactivity.

The postoperative cognitive dysfunction (POCD) is defined as new developed cognitive functional disorder after surgery and anesthesia. Symptoms are subtle and showing manifold pattern. Mechanisms leading to this entity are still not solved entirely. Experimental results showing immunological response of the innate immune system leading to a neuroinflammation. Activation of the inflammatory response and the TNF-α and NF-kB signal cascades are destroying the integrity of the blood-brain-barrier via excretion of different cytokines. This enables macrophages migration into the hippocampus and allows the disabling of brain memory response. Anti-inflammatory response could inhibit these proinflammatory action and dysfunction would be prohibited.

Quinoline acid (QUIN) has been shown to stimulate lipid peroxidation, production of reactive oxygen species, and mitochondrial dysfunction. Studies performed in organotypic cultures of rat corticostriatal system indicate that concentrations of QUIN even just slightly higher than physiological concentrations can cause neurodegeneration after a few weeks of exposure. Spinal neurons have been found to be especially sensitive to QUIN variations causing cell death with just nanomolar concentrations of this metabolite.

The kynurenine pathway (KP) metabolizes the essential aminoacid tryptophan and generates a number of neuroactive metabolites called the kynurenines. Segregated into at least two distinct branches, often termed as the "neurotoxic" and "neuroprotective" arms of the KP, they are regulated by the two enzymes kynurenine 3-monooxygenase (KMO) and kynurenine aminotransferase (KAT), respectively. Interestingly, several enzymes in the pathway are under tight control of inflammatory mediators and even small changes can cause major injuries. Recent years have seen a tremendous increase in our understanding of neuroinflammation in CNS disease. There is evidence, that neuroinflammation is linked to the innate immune system and the role of NAPLP3 inflammasomes. This could be an option of a protective therapeutic approach in these kind of disorders. This theory is supported by the fact that the increased τ-protein concentration in patients with ND-disease acts like alarmins. These alarmins are responsible for the activated innate response in sense of inflammation.

The present invention provides an in vitro method for the determination of a neurodegenerative disease wherein separately from each other the content of L-kynurenine (L-KYN) and kynurenic acid (KYNA) in a body fluid is determined and the quotient of the content of kynurenine to the content of kynurenic acid is calculated.

Neurodegenerative diseases in the sense of the present invention comprise in particular Alzheimer's disease, Parkinson's disease, vascular dementia, postoperative cognitive dysfunction and/or age-related depression.

There are many forms of mental impairment that can be designated in a slightly different manner. A clear borderline is, however, difficult to draw. Mental impairments comprise for example amnestic mild cognitive impairment that affects mainly the memory. Furthermore, nonamnestic mild cognitive impairment is also known whereby the memory is not strongly affected, but other mental capabilities are significantly reduced. For Alzheimer's disease there are several stages known and the classification into the several stages depends on the testing methods. It has been assumed that cognitive impairment, dementia and Alzheimer dementia may be gender-specific and dependent on the genetic heritance of the test groups (e.g. Caucasians vs. Afro-Americans). It seems that the mental impairment is definitely age-related. With increasing age the mental impairment increased significantly whereby the increase starts with the age of 60 to 70 years.

The in vitro test method of the present invention can be performed with a body fluid. Body fluid in the sense of the present invention is any liquid that can be derived from a human body. The most used liquid is serum or plasma. For neurodegenerative diseases the testing of liquor may also be suitable. To obtain a liquor sample is, however, difficult and sometimes dangerous. Therefore, other body fluids, which may be easily obtainable, are preferred. In a particularly preferred embodiment of the present invention the in vitro test method is performed with saliva since saliva can be easily obtained and surprisingly the test methods can be performed successfully in saliva.

A main aspect of the present invention is that diagnostic predictions can be made from the relation of kynurenine to kynurenic acid in the body fluid. In order to determine this relationship the same sample is tested with regard to the content of kynurenine and kynurenic acid.

The in vitro method of the present invention uses two different components, which specifically bind to kynurenine, and on the other hand to kynurenine acid whereby both components are usually located at different places of the test device in order to allow clear distinction of the test results.

The term "specifically binding" means that one component binds only to kynurenine (L-kynurenine) and not to kynurenic acid. The other component binds specifically only to kynurenic acid and not to L-kynurenine. Furthermore, both components should not bind to any other substance or impurity that may be present in the body fluid to be tested. The binding of the component to any impurity that may be present in the body fluid may influence the test result and is highly undesirable.

In a preferred embodiment as component that specifically binds to kynurenine or to kynurenic acid antibodies are used. Such antibodies can be prepared by immunizing a laboratory animal like for example rabbits, goats, horses or sheep and the polyclonal antibodies may be further purified and used in the test.

In an alternative embodiment the component may be a monoclonal antibody, which can be produced by the well-known hybridoma technology. Suitable clones are selected which show the desired binding pattern. When a suitable monoclonal antibody has been identified it is possible to sequence the binding regions and to prepare derivatives of the monoclonal antibody by genetic engineering. It is well-known for example to produce single chain antibodies or diabodies in order to name only a few. Such constructs contain an antigen binding region that fits perfectly to the structure of the target molecule.

In another preferred embodiment the components that bind specifically to L-kynurenine or to kynurenic acid, respectively, are aptamers. Aptamers are biocompatible molecules like DNA- or RNA-oligonucleotides or peptides, which enable specific targeting of molecules. Aptamers are for example oligonucleotides or peptides that have high sensitivity and robust selectivity towards several types of target molecules including small molecules like L-kynurenine or kynurenic acid, respectively. Usually the aptamers contain a variable loop and stem region that bind to a specific pocket or surface structure of the target molecules.

In preferred embodiments the aptamers are selected in vitro by using a process called "systematic evolution of ligands by exponential enrichment" (SELEX). In the course of a usual SELEX procedure the target (L-kynurenine or kynurenic acid) is brought into contact with a library of potential ligands. The candidates, which have the best binding characters, are separated and further improved by slight changes of the binding molecules and further selection of the better candidates. After several rounds of enrichment and improvement an aptamer may be obtained which has high specificity for the target molecules.

In addition thereto it may be advantageous to perform also a negative selection in order to make sure that the aptamer binds exclusively either to L-kynurenine or to kynurenic acid, respectively. In such negative selection those candidates are singled out which do not allow a clear distinction of the target.

In a preferred embodiment the in vitro method of the present invention is performed as an ELISA (Enzyme Linked Sorbent Test Assay).

There are different configurations of ELISA tests known. Usually a so-called sandwich ELISA test is performed. In such an ELISA test the compound that binds specifically to L-kynurenine or kynurenic acid is fixed on a solid surface (e.g. the bottom of a microtiter well). Unspecific binding sites are saturated (e.g. with skim milk powder) in order to avoid unspecific binding.

Preferably the ELISA test kit must contain separate entities (e.g. microtiter wells) with components that bind specifically to kynurenine only and in other entities components that bind to kynurenic acid only. Usually several microtiter wells are coated with the component in order to allow an easy dilution of the sample for a determination of the content of the analyte.

The binding of L-kynurenine or kynurenic acid, respectively, to the relevant wells is usually detected with another antibody that binds, however, to another area of the target molecule in order to avoid a negative interference of the binding. Such antibody is usually coupled with a signal generating means that may be for example an enzyme like horseradish peroxidase. The presence of the analyte to be detected can then be seen by adding a precursor molecule, which is converted to another molecule having different properties by the signal generating molecule. When for example in one wall kynurenine or kynurenic acid, respectively, is present the antibody binds to this molecule and with the activity of the signal generating means (e.g. horseradish peroxidase) a color signal is generated whereby the intensity is proportional to the amount of the bound target molecule (kynurenine or kynurenic acid). The reaction can be measured quantitatively and the amount of the analyte to be detected in the body fluid can be determined precisely.

In a further embodiment the in vitro test method is a lateral flow test. Lateral flow tests also known as Lateral Flow Immunochromatographic Assays are simple devices intended to detect the presence (or absence) of a target analyte sample without the need for specialized and costly equipment, though many lab based applications exist that are supported by a reading equipment. Typically, these tests are used for medical diagnostics either for home testing, point of care testing, or laboratory use. A widely spread and well known application is e.g. the home pregnancy test.

The technology is based on a series of capillary beds, such as pieces of porous paper or sintered polymer. Each of these elements has the capacity to transport fluid (e.g., saliva) spontaneously. The first element (the sample pad) acts as a sponge and holds an excess of sample fluid. Once soaked, the fluid migrates to the second element (conjugate pad) in which the manufacturer has stored the so called conjugate, a dried format of bio-active particles (see below) in a salt-sugar matrix that contains everything to guarantee an optimized chemical reaction between the target molecule (e.g., kynurenine) and its chemical partner (e.g., antibody) that has been immobilized on the particle's surface. While the sample fluid dissolves the salt-sugar matrix, it also dissolves the particles and in one combined transport action the sample and conjugate mix while flowing through the porous structure. In this way, the analyte binds to the particles while migrating further through the third capillary bed. This material has one or more areas (often called stripes) where a third molecule has been immobilized by the manufacturer. By the time the sample-conjugate mix reaches these strips, analyte has been bound on the particle and the third 'capture' molecule binds the complex. After a while, when more and more fluid has passed the stripes, particles accumulate and the stripe-area changes color. Typically there are at least three stripes: one (the control) that captures any particle and thereby shows that reaction conditions and technology worked fine, the second and third contains a specific capture molecule (compound specific for L-kynurenine and kynurenic acid, respectively) and only captures those particles onto which an analyte molecule has been immobilized. After passing these reaction zones the fluid enters the final porous material, the wick, that simply acts as a waste container. Lateral Flow Tests can operate as either competitive or sandwich assays.

In principle, any colored particle can be used, however, latex (blue color) or nanometer sized particles of gold (black / grey, red color) are most commonly used. The gold particles are red in color due to localized surface plasmon resonance. Fluorescent or magnetic labeled particles can also be used, however these require the use of an electronic reader to assess the test result.

The sample first encounters colored particles, which are labeled with antibodies raised to the target analyte. The test line will also contain antibodies to the same target, although it may bind to a different epitope on the analyte. The test line will show as a colored band in positive samples. An example of the sandwich assay is the sandwich ELISA.

While not strictly necessary, most test kits preferably incorporate a second line, which contains an antibody that picks up free latex/gold in order to confirm the test has operated correctly.

In a preferred embodiment the single components of the lateral flow assay are adapted in such a manner that the presence of kynurenine or kynurenic acid is indicated only when more than a certain threshold value of kynurenine is present in the sample.

A preferred test kit consists of the following components:
1. Sample pad - an absorbent pad onto the test sample (saliva) is applied
2. Conjugate or reagent pad A and B - this contains components (e.g. antibodies) specific to the target (kynurenine and kynurenic acid, respectively) analyte conjugated to colored particles (usually colloidal gold particles, or latex microspheres)
3. Reaction membrane - typically a hydrophobic nitrocellulose or cellulose acetate membrane onto which anti-target analyte antibodies are immobilized in a line across the membrane as a capture zone or test line (a control zone may also be present, containing antibodies specific for the conjugate antibodies)
4. Wick or waste reservoir - a further absorbent pad designed to draw the sample across the reaction membrane by capillary action and collect it.

The components of the strip are usually fixed to an inert backing material and may be presented in a simple dipstick format or within a plastic casing with a sample port and reaction window showing the capture and control zones.

There are two preferred embodiments of the test kits (lateral flow immunoassay) used in the method of the present invention:

### a. Double antibody sandwich assays

In this format the sample migrates from the sample pad through the conjugate pad where any target analyte present will bind to the conjugate. The sample then continues to migrate across the membrane until it reaches the capture zone where the target/conjugate complex will bind to the immobilized antibodies producing a visible line on the membrane. The sample then migrates further along the strip until it reaches the control zone, where excess conjugate will bind and produce a second visible line on the membrane. This control line indicates that the sample has migrated across the membrane as intended. Two clear lines on the membrane show a positive result. A single line in the control zone is a negative result. Double antibody sandwich assays are most suitable for larger analytes, such as bacterial pathogens and viruses, with multiple antigenic sites. For the present invention a suitable pair of antibodies must be selected which bind to different epitopes on kynurenine and kynurenic acid, respectively.

When the test methods or kits suitable for performing such method use antibodies which bind specifically to kynurenine, the term "antibody" means not only antibodies artificially produced for example by immunization of a laboratory animal like rabbit, sheep or goat. It comprises also in a preferred embodiment monoclonal antibodies produced according to the hybridoma technology. Moreover, the term "antibody" comprises also antigen-binding fragments of antibodies such as recombinantly produced antigen-binding fragments. Such constructs can be produced by phage display and technologies derived there from.

### b. Competitive assays

Competitive assays are primarily used for testing small molecules and differ from the double antibody sandwich format in that the conjugate pad contains antibodies that are already bound to the target analyte, or to an analogue of it. If the target analyte is present in the sample it will therefore not bind with the conjugate and will remain unlabelled. As the sample migrates along the membrane and reaches the capture zone an excess of unlabelled analyte will bind to the immobilized antibodies and block the capture of the conjugate, so that no visible line is produced. The unbound conjugate will then bind to the antibodies in the control zone producing a visible control line. A single control line on the membrane is a positive result. Two visible lines in the capture and control zones is a negative result. However, if an excess of unlabelled target analyte is not present, a weak line may be produced in the capture zone, indicating an inconclusive result. Competitive assays are most suitable for testing for small molecules, such as mycotoxins, unable to bind to more than one antibody simultaneously. There are a number of variations on lateral flow technology. The capture zone on the membrane may contain immobilized antigens or enzymes - depending on the target analyte - rather than antibodies. It is also possible to apply multiple capture zones to create a multiplex test.

Lateral flow immunoassays are simple to be used by untrained operators and generally produce a result within 15 minutes. They are very stable and robust, have a long shelf life and do usually not require refrigeration. They are also relatively inexpensive to produce. These features make them ideal for use at the point-of-care and for testing samples in the field, as well as in the laboratory. However, their sensitivity is limited without additional concentration or culture procedures. There are quantitative tests available, but our target is a qualitative test for saliva within a certain range. Therefore, the preferred test kit is adjusted to measure kynurenine only if present above a certain concentration. Below such concentration the test kit will show a negative result.

The method of the present invention is preferably performed with saliva. Saliva is a clinically informative, biological fluid that is useful for novel approaches to prognosis, laboratory or clinical diagnosis, and monitoring and management of patients. Saliva contains multiple biomarkers and an overview of the principles of salivary gland secretion, methods of collection, and discussion of general uses can be found in a report of a meeting published in the Annals of the New York Academy of Sciences Malamud D, Niedbala RS Oral-based diagnostics NY Acad Sci 2007; Boston Mass.

The present invention is based on determining the quotient of the presence of kynurenine compared to the presence of kynurenic acid in the body fluid. Therefore, the lateral flow test is designed to perform two measurements from the sample (preferably saliva) at the same time. In order to make the use of the test simple the test may be calibrated to certain contents of L-kynurenine or kynurenic acid, respectively. The lateral flow test is preferably designed in such a manner that when a critical concentration, which has been fixed, previously is reached a color signal can be seen. By using suitable dilutions it is possible to design the lateral flow test in such a manner that it can be easily seen whether the quotient of kynurenine to kynurenic acid is above 1 or below 1. Usually a quotient below 1 speaks for no neurodegenerative disease whereas a quotient above 1.0 speaks for a neurodegenerative disease.

In another embodiment the present invention provides suitable kits for performing the method according to the invention. Such a kit comprises preferably means for the determination of kynurenine and kynurenic acid, respectively, in saliva. Such means may work on different principles. It is possible to use a specific color reagent, which detects the presence of kynurenine and/or kynurenine derivatives. Alternatively the kit may comprise at least one or preferably two antibodies specifically binding to kynurenine or kynurenic acid. Preferably when two antibodies are used, such antibodies do not bind to the same epitope in order to allow the formation or a sandwich formed by the first antibody, kynurenine or its derivative and the second antibody.

In one embodiment of the present invention the determination of kynurenine and kynurenic acid is performed by a coloring reaction. The sample in the determination test is saliva. Before the content of kynurenine or derivatives thereof can be determined, components, which may negatively affect the correct, and precise test result have to be removed. In a preferred embodiment undesired components of saliva that may disturb the correct test result are removed preferably by precipitation of the components that disturb the result of the measurement. Such precipitation can preferably be performed by using trichloric acid. It is, however, possible to use other methods for deproteinization of saliva than using trichloric acid. After the disturbing components of saliva have been removed by precipitation it may be necessary to separate the phases by centrifugation. The supernatant is then preferably reacted with a coloring reagent that may preferably be Ehrlich's reagent. After development of the color the samples are measured by measuring the absorbance at a suitable wavelength. Preferably the test is performed in a quantitative or semi-quantitative manner. In the test method either a calibration curve can be used or a certain threshold value is fixed in the test kit in order to avoid false positive results.
Figure 1 shows a schematic overview of the kynurenine pathway, the major route of tryptophan degradation in higher eukaryotes. Enzymes are indicated in italics. The neurotoxic metabolites QUIN and 3-HK are shown as well as the neuroprotective metabolite KYNA.
Figure 2 shows L-kynurenine and kynurenic acid concentrations in µM for normal controls and differences between <60 and >60m years of age. Measurements were made in serum and saliva as well.
Figure 3 shows a comparison of kynurenine (measured in serum) in 2 groups: controls (n=194); patients with neurodegenerative disorders (n=42). There was a significant difference for kynurenine and kynurenine acid (p<0.001).
Figure 4 shows values given for the correlation between serum and saliva in Kynurenine and Kynurenine-acid measurement.

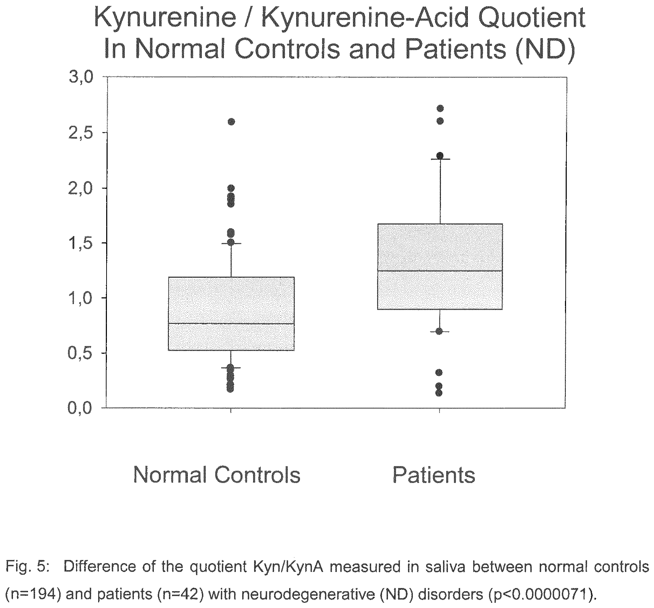

Figure 5 shows the difference of the quotient Kyn/KynA measured in saliva between normal controls (n=194) and patients (n=42) with neurodegenerative (ND) disorders (p<0.0000071).

The present invention is described in more detail in the Figure and the following Examples.

### Example 1

Forty-two patients with cerebral dementia (mean age 71 + 5,3 years, mean MMS-score 22) were enrolled in a comparative study with normal controls (n=194; mean age 48,8 years, range 16-88 years). Aim of our study was to detect changes in the tryptophan metabolism in patients with cerebral dementia, by estimating either kynurenine, kynurenic acid and ratio of kyn/kynA in plasma and in saliva. There was no age related difference between a group I of normal controls (n=93, **age>60,** mean age 71,3 years, range 60-88) and group II (n=101, **age<60,** mean age 38,8, range 16-60; Fig. 2). This is demonstrating that the disease must not necessarily be age related in general. The neurodegenerative diseases may be caused by different reasons whereby, however, the frequency of neurodegenerative diseases increases statistically with increasing age.

### Example 2

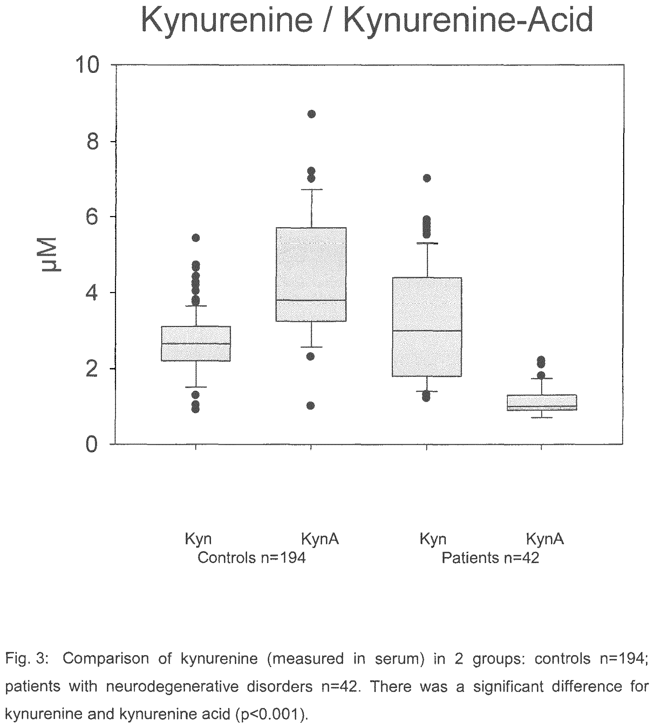

Kynurenine was significantly higher and kynurenic acid lower and ratio was different in patients with neurodegenerative disorders. This could be demonstrated in serum as well as in saliva. The measured values are shown in Fig. 3.

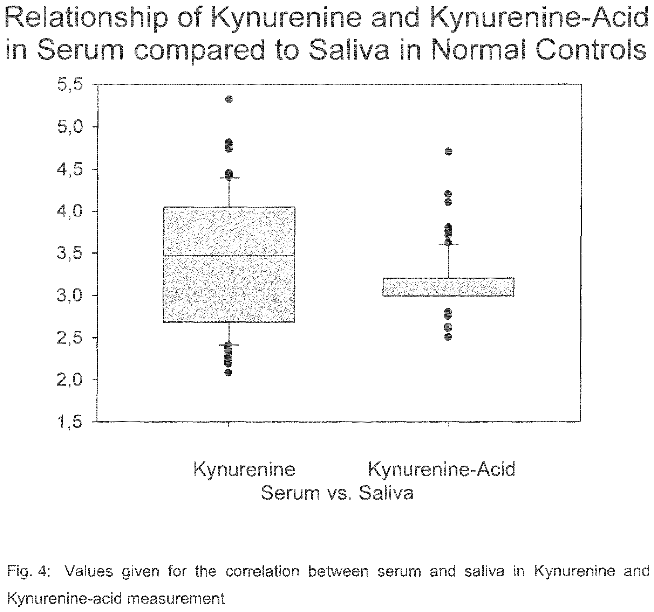

There was a correlation between the values of kynurenine and kynurenic acid in serum compared to saliva (saliva 1: 3,5 in serum for kynurenine and 1:3,2 in saliva for kynurenic acid in normal controls, Fig. 4).

### Example 3

Patients with neurodegenerative disease showed a total different pattern: mean values for kynurenine in serum as well as in saliva were significant higher (4,80 ± 0,6 µM for serum and 1,34 + 0,3 µM for saliva) whereas values for kynurenic acid were significant lower (1,58 ± 0,3 in serum and 1,3 ± 0,2 in saliva, Fig. 3). This is in correspondence with the theory of the pathophysiology of the disease: the neuro-protective part (kynurenic acid) is downregulated and the inflammatory part (kynurenine) is upregulated.

### Example 4

Concerning the measurement of kynurenine and kynurenic acid in serum and saliva we could demonstrate the correlation for serum and saliva. The small numeric difference between both values is related to the different method of measurement.

We could demonstrate that measurement of kynurenine and kynurenic acid is possible in serum as well as in saliva. There is a relationship between the values in serum compared to the values in saliva.

Compared to the data evaluated in normal controls, data in patients showed significant different pattern and could be easy identified.

In total, already in this small group of patients it could be demonstrated, that kynurenine/ kynurenic acid measurement is a tool to identify cerebral disorders as well as to monitor them. The measured kynurenine / kynurenic acid quotient is a clear indicator for neurodegenerative diseases if the quotient is 1.0 or higher.

## Claims

1. An in vitro method for the determination of a neurodegenerative disease wherein separately from each other the content of kynurenine and kynurenic acid in a body fluid is determined and the quotient of the content of kynurenine to the content of kynurenic acid is calculated.

2. In vitro method according to claim 1 wherein the neurodegenerative disease is selected from the group consisting of Alzheimer's disease, Parkinson's disease, vascular dementia, postoperative cognitive dysfunction and/or age-related depression.

3. An in vitro method according to claim 1 or 2 that allows monitoring of therapeutic interventions in neurodegenerative diseases.

4. In vitro method according to claims 1 to 3 **characterized in that** the body fluid is serum or saliva.

5. In vitro test method according to any of claims 1 to 4 **characterized in that** it comprises at least one component that specifically binds to kynurenine and at least one component, which specifically binds to kynurenic acid.

6. In vitro method according to claim 5 **characterized in that** the component which specifically binds to kynurenine is spatially separately localized from the component which specifically binds to kynurenic acid.

7. In vitro method according to claims 4 to 6 wherein at least one component is an antibody.

8. Method according to claim 4 to 6 **characterized in that** at least one component is an aptamer.

9. In vitro method according to any of claims 1 to 7 **characterized in that** it is an ELISA test.

10. In vitro method according to any of claims 1 to 7 **characterized in that** it is a lateral flow test.

11. Test kit for performing a method according to any of claims 1 to 9 **characterized in that** it is an ELISA test kit.

12. Test kit according to any of claims 1 to 7 and claim 10 **characterized in that** it is a lateral flow test.
